# EUROPÄISCHE PATENTSCHRIFT

(11) **EP 2 032 095 B1**
(45) Veröffentlichungstag und Bekanntmachung des Hinweises auf die Patenterteilung: **18.01.2012**
(21) Anmeldenummer: 07722539.9
(22) Anmeldetag: 18.06.2007
(51) Int. Cl.: A61F 5/41

(54) **EXTENSIONSGERÄT ZUR DAUERHAFTEN PENISVERGRÖSSERUNG UND -BEGRADIGUNG**
EXTENSION APPARATUS FOR PERMANENTLY ENLARGING AND STRAIGHTENING A PENIS
APPAREIL D'EXTENSION PERMETTANT D'AGRANDIR ET DE REDRESSER LE PÉNIS DE FAÇON PERMANENTE

(30) Priorität: 26.06.2006 DE 102006029258; 01.06.2007 DE 102007026063
(43) Veröffentlichungstag der Anmeldung: 11.03.2009
(73) Patentinhaber: MSP Concept GmbH & Co. KG, 10719 Berlin (DE)
(72) Erfinder: RUDI, Johann, 51545 Waldbröl (DE)
(74) Vertreter: Specht, Volker
(86) Internationale Anmeldenummer: PCT/DE2007/001097
(87) Internationale Veröffentlichungsnummer: WO 2008/000226

(56) Entgegenhaltungen:
- EP-B1- 1 023 013
- CA-A1- 2 444 663
- DE-A1- 10 001 331
- DE-U1-202005 017 165
- FR-A- 1 605 238

## Beschreibung

Die Erfindung betrifft ein Extensionsgerät zur dauerhaften Penisvergrößerung und -begradigung durch Langzeitdehnung, das ein am Penis angebrachtes Befestigungsmittel und eine mit diesem verbundene Zugvorrichtung umfasst.

Ein Penisextensionsgerät der oben erwähnten Art ist beispielsweise in der EP 1 023 013 beschrieben. Das Zugmittel zur Ausübung einer Zugwirkung am vorderen Ende des Penis ist hier ein an der Peniswurzel mittels einer Ringmanschette gehaltenes elastisches oder längenverstellbares flexibles Band, das um den Körper des Benutzers gelegt wird und dessen freies Ende mit einem Befestigungsmittel in Form eines schalenförmigen Objektträgers und eines Bändchens mit dem Penis verbunden ist. Aufgrund der von dem an der Peniswurzel als Widerlager gehaltenen Band ausgeübten Zugkraft an der Penisspitze wird der Penis gedehnt und dabei dauerhaft vergrößert'und gegebenenfalls begradigt. Diese Art der Befestigung erweist sich als in mehrfacher Hinsicht nachteilig. Insbesondere kann es bei den üblicherweise anzuwenden Zugkräften zu schmerzhaften Druckstellen kommen, so dass eine dauerhafte Dehnungsbehandlung nicht gewährleistet ist.

Ein aus der DE 100 01 331 A1 bekanntes Befestigungsmittel umfasst ein vollständig über die Glans penis stülpbares, kondomartiges zylindrisches Hütchen aus hochelastischem Material, das in seinem vorderen Bereich ein Anschlusselement zur Kopplung mit dem Zugmittel und in seinem hinteren Bereich einen zur dichten Anlage am Penisschaft unmittelbar hinter der Glans penis geeigneten, als Dichtungselement wirkenden Ringabschnitt aufweist. Aufgrund der am vorderen Bereich angreifenden Zugkraft und der Abdichtung am Penisschaft soll ein Unterdruck entstehen, aufgrund dessen und der elastischen Ausbildung sowie einer zusätzlichen Klebebeschichtung bzw. eines doppelseitigen Klebebandes das Befestigungsmittel sicher und schonend am Penis gehalten ist. Bei dem aufgrund des Klebemittels mit erheblichem Aufwand anzulegenden Befestigungsmittel bewirkt eine durch den Unterdruck erzielte Volumenvergrößerung der Glas penis eine hohe Flächenpressung an deren Umfang und damit eine Verschlechterung der Durchblutung. Die kraftschlüssige, durch hohe Reib- und Klebekräfte bewirkte Befestigung am Penis führt zu Schwellungen und in deren Folge zum Austreten von Lymphflüssigkeit aus dem Gewebe.

Verfahren zum Strecken und Dehnen des Penis durch Aufbringen einer Zugkraft und damit zur Erzielung eines Größenwachstums werden im Übrigen seit langem angewendet, beispielsweise bei Naturvölkern durch Anhängen von Gewichten.

Der Erfindung liegt die Aufgabe zugrunde, ein Extensionsgerät zur Penisvergrößerung und -begradigung zu entwickeln, das einfach ausgebildet ist und mit geringem Aufwand angelegt werden kann und eine sichere und schonende Befestigung am Penis sowie einen hohen Tragekomfort gewährleistet.

Erfindungsgemäß wird die Aufgabe mit einem gemäß den Merkmalen des Patentanspruchs 1, 2 oder 3 ausgebildeten Penisextensionsgerät gelöst. Vorteilhafte Weiterbildungen der Erfindung ergeben sich aus den Unteransprüchen.

Der Grundgedanke der Erfindung besteht darin, dass das Befestigungsmittel zur Übertragung der von einer Zugvorrichtung ausgeübten Zugkraft auf den Penis einen die Glans penis formschlüssig aufnehmenden, formstabilen, im Wesentlichen starren Aufnahmekörper umfasst, dessen Innenkontur im Wesentlichen der Form der Glans penis entspricht und der über ein Verschlussventil an eine Pumpe zur Erzeugung eines Luftdruckunterschieds, und zwar durch Zuführen bzw. Absaugen von Luft beim Anlegen bzw. Einführen der Glans penis und beim Halten im Aufnahmekörper bei geschlossenen Verschlussventil unter einem geringen Unterdruck, anschließbar ist und an dessen zur Einführung der Glans penis vorgesehene Öffnung eine Schlauchdichtung anschließt.

Aufgrund der formschlüssigen, im Wesentlichen vollflächigen Verbindung zwischen Befestigungsmittel und Glans penis kann eine hohe und langanhaltende, aber dennoch schonende Kraftwirkung ausgeübt werden, ohne dass gesundheitliche Schäden, Druckstellen oder Schmerzen zu befürchten sind. Der Unterdruck wirkt zunächst zur Erleichterung des Einführens, aber auch während der Dehnungsbehandlung derart, dass das Befestigungsmittel ständig dicht an der gesamten Oberfläche der Glans penis anliegt, so dass schon bei geringem Unterdruck eine gleichmäßige Haltekraft nur an der Glans penis wirkt und infolge des geringen Unterdrucks und des an die Größe und Form der Glans penis angepassten Befestigungsmittels keine Überdehnung des Gewebes mit den damit verbundenen Nebenwirkungen erfolgt. Der Unterdruck ist lediglich so groß, dass das in Form und Größe angepasste Befestigungsmittel tatsächlich vollflächig an der Glas penis anliegt, um durch Formschluss und Adhäsion eine sichere und schonende Verbindung mit der Glans penis herzustellen. Die Vorrichtung kann auf einfache Weise und bequem angelegt werden und gewährleistet einen hohen Tragekomfort. Da zur Halterung lediglich die Glans penis benötigt wird, ist das Gerät vorteilhaft auch zur Anwendung bei sehr kurzen Penissen geeignet. Entsprechend der unterschiedlichen Form und Größe der Glans penis sind bezüglich der Innenkontur unterschiedlich ausgebildete Aufnahmekörper vorgesehen.

Andererseits ist es möglich, bei Verwendung nur eines einheitlich ausgebildeten Aufnahmekörpers an die Form und Größe des Aufnahmekörpers angepasste Einlagen vorzusehen, die in den Aufnahmekörper eingesetzt werden können und die auch selbsttätig anpassend ausgebildet sein können.

Gemäß einem weiteren wichtigen Merkmal der Erfindung kann die Öffnung zum Einführen der Glans penis in den Aufnahmekörper mit einer elastischen Membran verschlossen sein, die im Zuge des Einführens der Glans penis unter Unterdruck die Glans penis vollflächig umschließt und unabhängig von deren Form und Größe den vollständigen und schonend wirkenden Formschluss mit der Glans penis innerhalb des Aufnahmekörpers herstellt.

Die Membran kann aufgrund ihrer Elastizität während der Benutzung auftretende individuelle Änderungen der Form und Größe der Glans penis ausgleichen, so dass in jedem Fall eine gleichbleibende Haltewirkung gewährleistet ist.

In weiterer Ausbildung der Erfindung ist an den Innenflächen der Schlauchdichtung und des Aufnahmekörpers bzw. der Einlage oder der Membran ein Gleitmittel vorgesehen, das zum einen dem leichten Einführen dient und zum anderen eine besonders innige, adhäsiv wirkende Verbindung zwischen Glans penis und Aufnahmekörper bzw. Einlage oder Membran schafft.

Gemäß einem noch anderen Erfindungsmerkmal ist an dem freien Ende der am Aufnahmekörper befestigten Schlauchdichtung ein Stützring angebracht, dessen Innendurchmesser etwa entsprechend dem Penisdurchmesser dimensioniert ist. Das erleichtert das Einführen der Glans penis und verhindert ein Hineinrutschen der Schlauchdichtung in den Aufnahmekörper.

An den Enden der Schlauchdichtung kann zu deren sicherer Befestigung am Aufnahmekörper und am Stützring eine elastische Haltewulst ausgebildet sein.

Die Schlauchdichtung und die Membran können aus der schlauchartigen Tülle bzw. dem Ballonabschnitt eines herkömmlichen Luftballons entsprechender Größe hergestellt werden.

Zur Erzeugung des Unterdrucks dient ein am Aufnahmekörper angeformter, mit einem Verschlussventil versehener Luftstutzen. Über das Verschlussventil kann, beispielsweise mit einer Pumpe, Luft dosiert abgesaugt werden.

Bei einer in Verbindung mit dem Aufnahmekörper verwendeten Membran kann unmittelbar vor dem Einführen der Glans penis durch Zuführen von Luft über den Luftstutzen in den Aufnahmekörper eine elastische Dehnung (Aufwölbung) der Membran zur Verbesserung des Kontaktes mit der Glans penis erfolgen, um dadurch einen Kontakt ohne Lufteinschlüsse zwischen Glans penis und Membran zu gewährleisten.

Die zur Ausübung einer am Aufnahmekörper wirkenden Zugkraft erforderliche Zugvorrichtung umfasst ein längenverstellbares oder elastisches Spannband, das einerseits mit dem Aufnahmekörper, beispielsweise ein in das Verschlussventil integriertes Kopplungselement, und andererseits über Befestigungsclips oder unmittelbar mit einem am Körper des Benutzers fixierten Gurt oder Träger, einem Gürtel, einem Hosenträger oder einem anderen Teil der Bekleidung verbunden wird. Auch das Anhängen eines Gewichts am Aufnahmekörper ist denkbar. Die Zugkraft am Aufnahmekörper kann auch über ein am Körper des Benutzers abgestütztes längenverstellbares Gestänge ausgeübt werden.

Ein Ausführungsbeispiel der Erfindung wird anhand der Zeichnung näher erläutert. Es zeigen:

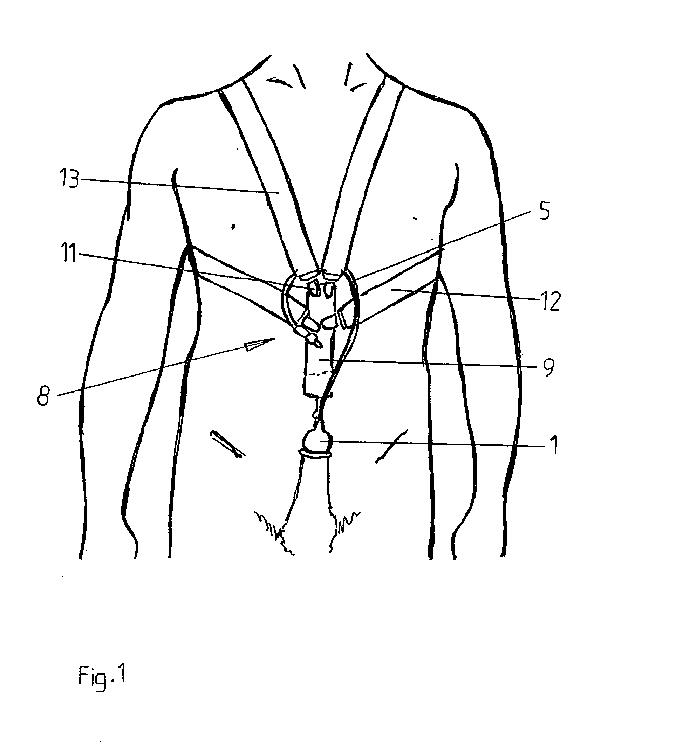

- Fig. 1: ein am Benutzer angelegtes, aus einer Zugvorrichtung und einem Befestigungsmittel bestehendes Extensionsgerät;
- Fig. 2: eine detaillierte Darstellung des Extensionsgerätes nach Fig. 1; und
- Fig. 3: eine vergrößerte Darstellung des Befestigungsmittels gemäß Fig. 2.

Das Befestigungsmittel 1 umfasst einen aus einem im wesentlichen starren Material bestehenden, die Glans penis vollständig umgebenden Aufnahmekörper 2, dessen Innenkontur im Wesentlichen der Form und Größe der gut durchbluteten Glans penis entspricht. Der Aufnahmekörper 2 hat eine zum Penisschaft hin weite Öffnung 3 und auf der dieser gegenüberliegenden Seite einen Luftstutzen 4, an den eine flexible Luftleitung 5 mit an deren freiem Ende angebrachtem Verschlussventil 6 angeschlossen ist. An der Außenseite des Aufnahmekörpers 2 ist außerdem eine der Öffnung 3 im Wesentlichen gegenüberliegende Zugöse 7 zur Herstellung der Verbindung zwischen dem Befestigungsmittel 1, das heißt dem Aufnahmekörper 2, und dem eine Zugkraft auf den Aufnahmekörper 2 und damit auf den Penis ausübenden Zugmittel 8 angebracht.

Das Zugmittel 8 umfasst ein flexibles - elastisches oder längenverstellbares - Spannband 9, das einerseits, hier über einen Karabinerhaken 10 und die Zugöse 7, mit dem Aufnahmekörper 2 und andererseits über Befestigungsclips 11 mit einem am Körper des Benutzers gehaltenen Gurt 12 oder Trägern 13 verbunden ist. Die Befestigung des Spannbandes 9 kann gleichermaßen an einem Gürtel oder Hosenträgern, gegebenenfalls auch an einem Kleidungsstück (jeweils nicht dargestellt), erfolgen. Es ist aber auch denkbar, dass die Zugwirkung am Aufnahmekörper 2 von einem an der Zugöse 7 befestigten Gewicht (nicht dargestellt) erzeugt wird.

Im Bereich der Öffnung 3 ist der Aufnahmekörper 2 so geformt, dass an dessen Außenumfang eine umlaufende Haltenut 14 zur Befestigung einer Schlauchdichtung 15 gebildet ist. Der im Innern des Aufnahmekörpers 2 an die Öffnung 3 anschließende, leicht schräg verlaufende Kantenbereich stellt eine umlaufende Anschlagfläche 20 dar, die bei Benutzung des Extensionsgerätes an der am unteren Rand der Glans penis gebildeten umlaufenden Fläche angreift. Dadurch wird eine formschlüssige Verbindung zwischen dem Aufnahmekörper 2 und der in diesem aufgenommenen Glans penis hergestellt, die eine sichere Kraftübertragung durch Formschluss (und nicht durch Haftreibung) gewährleistet.

In der Haltenut 14 des Aufnahmekörpers 2 ist das eine Ende der aus einem elastischen Material bestehenden, an den Enden jeweils eine Haltewulst aufweisenden Schlauchdichtung 15 elastisch gehalten. Das andere Ende der Schlauchdichtung 15 ist in der Umfangsnut 16 eines Stützrings 17 fixiert.

Bei der Benutzung des Extensionsgerätes wird die Glans penis über die Schlauchdichtung 15 in den Aufnahmekörper 2 eingeführt, wobei auf deren Innenfläche - wie im Übrigen zur Verbesserung der Adhäsionswirkung auch auf der weiter unten beschriebenen Membran 18 bzw. im Aufnahmekörper 2 - ein Gleitmittel zur Erleichterung des Einführens aufgetragen werden kann. Der im Durchmesser etwa entsprechend dem Penisumfang dimensionierte Stützring 17 ermöglicht ein einfaches Einführen der Glans penis und verhindert zudem, dass die Schlauchdichtung 15 in den Aufnahmekörper 2 rutscht. Während des Einführens wird zudem durch Luftabsaugen am Verschlussventil 6 ein leichter Unterdruck im - mittels der Schlauchdichtung 15 abgedichteten - Aufnahmekörper 2 erzeugt, der das Einführen der Glans penis bis zu deren Anliegen an der Innenkontur des Aufnahmekörpers 2 erleichtert. Die Außenfläche der Glans penis liegt jetzt - vorzugsweise unter Zwischenschaltung des oben erwähnten Gleitmittels - vollflächig an der Innenfläche des beidseitig mittels Verschlussventil 6 und Schlauchdichtung 15 dicht verschlossenen Aufnahmekörpers 2 an, so dass eine formschlüssige Verbindung zwischen der Glans penis und dem Aufnahmekörper 2 besteht und die am Aufnahmekörper 2 wirkenden Zugkräfte sicher, gleichmäßig und schonend, vorzugsweise über ein hautfreundliches, adhäsiv wirkendes Gleitmittel, auf die Glans penis und somit auf den Penis übertragen werden können. Da die Oberfläche der Glans penis überall an der Innenkontur des Aufnahmekörpers 2 anliegt und nur ein geringer Unterdruck zur Wahrung der Formschlüssigkeit an der Glans penis wirkt, werden unerwünschte Nebenwirkungen verhindert. Der Unterdruck dient nur zum leichteren Einführen der Glans penis und zu deren formschlüssigem - durch Adhäsionskräfte aufgrund des eingesetzten Gleitmittels verbessertem - Kontakt mit der Innenfläche des Aufnahmekörpers 2. Der Unterdruck wirkt jedoch nicht als primäre Haltekraft.

Form und Größe der Glans penis können sich bei unterschiedlichen Benutzern unterscheiden, so dass - sofern keine Membran verwendet wird - dementsprechend Aufnahmekörper 2 oder Einlagen in verschienenen Größen bereitgehalten werden sollten, um bei geringem Unterdruck, der nicht unmittelbar an der Glans penis wirkt, einen vollflächigen formschlüssigen Kontakt mit der Glans penis zu gewährleisten.

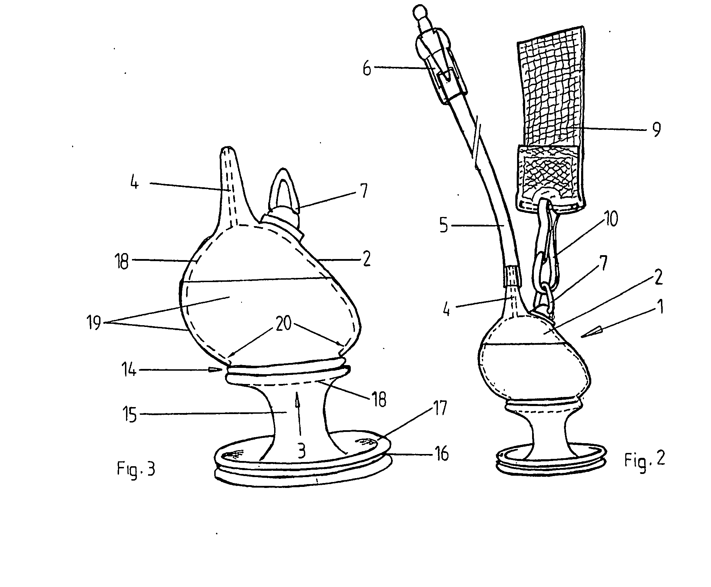

Wie die Figuren 2 und 3 zeigen, kann jedoch unabhängig von der jeweiligen Größe der Glans penis mit ein und demselben Aufnahmekörper 2, jedoch unter Verwendung einer elastisch verformbaren Membran 18, dennoch eine die Glans penis vollflächig kontaktierende Innenkontur des Aufnahmekörpers 2 erzielt werden. Die Membran 18 wird von einem über den Aufnahmekörpers 2 gestülpten und dabei dessen Öffnung 3 verschließenden Gummiballon 19 gebildet. Erst nach dem Überstülpen des Gummiballons 19 wird die Schlauchdichtung 15 am Aufnahmekörper 2 angebracht. Vor dem Einführen der Glans penis wird die Membran 18 durch kurzzeitige Luftzufuhr über das Verschlussventil 6 in den Aufnahmekörper 2 nach außen aufgewölbt, um während des Einführens einen innigen Kontakt mit der Glans penis zu bewirken. Beim Einführen der Glans penis unter gleichzeitiger Erzeugung eines Unterdrucks durch Luftabsaugen am Verschlussventil 6 mittels einer Pumpe (nicht dargestellt) wird die Membran 18 über der Öffnung 3 - zusammen mit der Glans penis und sich an deren Oberfläche anlegend - in den Aufnahmekörper 2 eingesaugt. Dadurch ist gewährleistet, dass eine Glans penis, die auch deutlich kleiner als das Innenvolumen des Aufnahmekörpers 2 sein kann und auch eine von dessen Innenkontur abweichende Form haben kann, an ihrer gesamten Außenfläche, vorzugsweise unter Zwischenschaltung eines hier adhäsiv wirkenden Gleitmittels, vollflächig umschlossen und formschlüssig in dem Aufnahmekörper 2 fixiert ist, und zwar ohne dass der beim Einführen erzeugte Unterdruck unmittelbar an der Glans penis wirkt.

Der Aufnahmekörper 2 mit Luftstutzen 4 ist einstückig aus starren oder im Wesentlichen starrem Material, beispielsweise Kunststoff, Hartgummi oder dgl., geformt.

### Bezugszeichenliste

- 1: Befestigungsmittel
- 2: Aufnahmekörper
- 3: Öffnung v. 2
- 4: Luftstutzen
- 5: Luftleitung
- 6: Verschlussventil
- 7: Zugöse
- 8: Zugvorrichtung
- 9: Spannband v. 8
- 10: Karabinerhaken
- 11: Befestigungsclip
- 12: Gurt
- 13: Träger
- 14: Haltenut v. 2
- 15: Schlauchdichtung
- 16: Umfangsnut v. 17
- 17: Stützring
- 18: Membran
- 19: Gummiballon
- 20: umlaufende Anschlagfläche

## Patentansprüche

1. Extensionsgerät zur dauerhaften Penisvergrößerung und -begradigung durch Langzeitdehnung, das ein am Penis anzuordnendes Befestigungsmittel und eine mit diesem verbundene Zugvorrichtung umfasst, **dadurch gekennzeichnet, dass** das Befestigungsmittel einen die Glans penis weitgehend vollflächig formschlüssig aufnehmenden formstabilen Aufnahmekörper (2) aufweist, dessen Innenkontur weitgehend der Form der Glans penis entspricht, *wobei entsprechend der Form und Größe der Glans penis unterschiedlich ausgebildete Aufnahmekörper (2) vorgesehen sind,* und der an ein Mittel zum Zuführen und Absaugen von Luft zur Erzeugung eines Über- oder Unterdrucks anschließbar ist und an dessen zur Einführung der Glans penis vorgesehene Öffnung (3) eine elastische Schlauchdichtung (15) anschließt.

2. Extensionsgerät zur dauerhaften Penisvergrößerung und -begradigung durch Langzeitdehnung, das ein am Penis anzuordnendes Befestigungsmittel und eine mit diesem verbundene Zugvorrichtung umfasst, **dadurch gekennzeichnet, dass** das Befestigungsmittel einen die Glans penis weitgehend vollflächig formschlüssig aufnehmenden formstabilen Aufnahmekörper (2) aufweist, dessen Innenkontur weitgehend der Form der Glans penis entspricht, *indem entsprechend der jeweiligen Form und Größe der Glans penis unterschiedlich ausgebildete Einlagen in den Aufnahmekörper* (2) *einbringbar sind, die an die Innenfläche des Aufnahmekörpers und an die Außenkontur der Glans penis angeposst oder selbsttätig anpassend ausgebildet sind,* und der an ein Mittel zum Zuführen und Absaugen von Luft zur Erzeugung eines Unter- oder Überdrucks anschließbar ist und an dessen zur Einführung der Glans penis vorgesehene Öffnung (3) eine elastische Schlauchdichtung (15) anschließt.

3. Extensionsgerät zur dauerhaften Penisvergrößerung und -begradigung durch Langzeitdehnung, das ein am Penis anzuordnendes Befestigungsmittel und eine mit diesem verbundene Zugvorrichtung umfasst, **dadurch gekennzeichnet, dass** das Befestigungsmittel einen die Glans penis weitgehend vollflächig formschlüssig aufnehmenden Aufnahmekörper (2) aufweist, dessen Innenkontur weitgehend der Form der Glans penis entspricht, *indem über der Öffnung (3) des Aufnahmekörpers (2) eine elastische, unter der Wirkung eines Überdrucks nach außen aufwölbbare und unter der Wirkung eines Unterdrucks beim Einführen der Glans penis in den Aufnahmekörper einstülpbare Membran (18) angebracht ist, die die Glans penis unabhängig von ihrer jeweiligen Form und Größe vollflächig formschlüssig umschließt,* und der an ein Mittel zum Zuführen und Absaugen von Luft zur Erzeugung des Unter- oder Überdrucks anschließbar ist und an dessen zur Einführung der Glans penis vorgesehene Öffnung (3) eine elastische Schlauchdichtung (15) anschließt.

4. Extensionsgerät nach einem der Ansprüche 1 bis 3, **dadurch gekennzeichnet, dass** sich im Benutzungszustand an der Innenfläche des Aufnahmekörpers (2) oder der Einlage oder der Membran (18) ein Gleitmittel zur Unterstützung der innigen, adhäsiv wirkenden Verbindung mit der Glans penis befindet.

5. Extensionsgerät nach Anspruch 1, **dadurch gekennzeichnet, dass** an der Innenfläche der Schlauchdichtung (15) ein Gleitmittel zum Erleichtern des Einführens der Glans penis vorgesehen ist.

6. Extensionsgerät nach Anspruch 5, **dadurch gekennzeichnet, dass** das vom Aufnahmekörper (2) entfernte freie Ende der Schlauchdichtung (15) mit einem etwa entsprechend dem Penisdurchmesser dimensionierten Stützring (17) zum einfachen Einführen der Glans penis verbunden ist.

7. Extensionsgerät nach Anspruch 1 oder 6, **dadurch gekennzeichnet, dass** die Schlauchdichtung (15) an den Enden in einer Umfangsnut (16) des Stützringes (17) und in einer am Aufnahmekörper (2) gebildeten Haltenut (14) elastisch fixiert ist.

8. Extensionsgerät nach 7, **dadurch gekennzeichnet, dass** die Schlauchdichtung (15) an mindestens einem Ende eine elastische Haltewulst aufweist.

9. Extensionsgerät nach Anspruch 1, **dadurch gekennzeichnet, dass** die Schlauchdichtung (15) aus einem Tüllenabschnitt und die Membran (18) aus einem über den Aufnahmekörper (2) gestülpten Ballonabschnitt eines handelsüblichen Luftballons entsprechender Größe gebildet ist.

10. Extensionsgerät nach Anspruch 1, **dadurch gekennzeichnet, dass** das Mittel zum Zuführen und Absaugen von Luft ein am Aufnahmekörper (2) vorgesehenes Verschlussventil (6) umfasst, an das eine Luftsaug- und Druckpumpe anschließbar ist.

11. Extensionsgerät nach Anspruch 1, **dadurch gekennzeichnet, dass** an der der Öffnung (3) gegenüberliegenden Seite des Aufnahmekörpers (2) ein Kupplungselement vorgesehen ist, das an eine Zugvorrichtung (8) koppelbar ist.

12. Extensionsgerät nach Anspruch 11, **dadurch gekennzeichnet, dass** das Kopplungselement in das Verschlussventil (6) integriert ist.

13. Extensions gerät nach Anspruch 1, **dadurch gekennzeichnet, dass** die Zugvorrichtung (8) von einem mit dem Aufnahmekörper (2) verbundenen, am Körper des Benutzers gehaltenen und als Widerlager dienenden Träger, Gurt, Gürtel oder sonstigem Bekleidungsaccessoire oder Bekleidungsstück oder auch einem stationären Objekt oder einem am Körper abgestützten längenverstellbaren Gestänge oder einem am Aufnahmekörper angehängten Gewicht gebildet ist.

## Claims

1. An extension device for permanent penis enlargement and straightening through long-term stretching, comprising a fastening element to be attached to the penis and a tensioning device connected thereto, **characterised in that** the fastening element has a receptacle (2) which largely fully accommodates the glans penis in a tight fitting and stable manner and the inner contour of which largely corresponds to the shape of the glans penis, whereby *depending* on the shape *and size of* the *glans penis differently designed receptacles (2)* are *envisaged,* and which can be connected to a means of supplying and extracting air to produce an overpressure or vacuum, with an elastic tubular seal (15) connecting to the opening (3) provided for inserting the glans penis.

2. An extension device for permanent penis enlargement and straightening through long-term straightening comprising a fastening element to be attached to the penis and a tensioning device connected thereto, **characterised in that** the fastening element has a receptacle (2) which largely fully accommodates the glans penis in a tight fitting and stable manner and the inner contour of which largely corresponds to the shape of the glans penis, *whereby depending on the shape and size of the glans penis variously designed inserts can be inserted into the receptacle (2) which are adapted to the inner surface of the receptacle and to the outer contour of the glans penis or are designed to adjust automatically thereto*, and which can be connected to a means of supplying and extracting air to produce an overpressure or vacuum, with an elastic tubular seal (15) connecting to the opening (3) provided for inserting the glans penis.

3. An extension device for permanent penis enlargement and straightening through long-term straightening comprising a fastening element to be attached to the penis and a tensioning device connected thereto, **characterised in that** the fastening element has a receptacle (2) which largely fully accommodates the glans penis in a tight fitting manner and the inner contour of which largely corresponds to the shape of the glans penis, *whereby via the opening (3) of the receptacle (2) an elastic membrane (18), which bulges outwards under the effect of* an overpressure and under *the effect of a vacuum turns inwards when introducing the glans penis into the receptacle, is attached which tightly surrounds the entire glans penis irrespective of its shape and size*, and which can be connected to a means of supplying and extracting air to produce an overpressure or vacuum, with an elastic tubular seal (15) connecting to the opening (3) provided for inserting the glans penis.

4. The extension device in accordance with any one of claims 1 to 3 **characterised in that** when in use there is a lubricant on the inner surface of the receptacle (2) or the insert or the membrane (18) to support the inner, adhesively acting connection with the glans penis.

5. The extension device in accordance with claim 1 **characterised in that** on the inner surface of the tubular seal (15) a lubricant is provided to facilitate the insertion of the glans penis.

6. The extension device in accordance with claim 5 **characterised in that** free end of the tubular seal (15) away from the receptacle (2) is connected to a support ring (17) measuring approximately the size of the diameter of the penis for simple insertion of the glans penis.

7. The extension device in accordance with claim 1 or 6 **characterised in that** the tubular seal (15) is elastically attached at the ends in a circumferential groove (16) of the support ring (17) and a retaining groove (14) formed on the receptacle (2).

8. The extension device in accordance with claim 7 **characterised in that** the tubular seal (15) has an elastic retaining bead on at least one end.

9. The extension device in accordance with claim 1. **characterised in that** the tubular seal (15) is formed of a sleeve section and the membrane (18) of a balloon section of a commercially available air balloon of appropriate size folded over the receptacle (2).

10. The extension device in accordance with claim 1 **characterised in that** the means of supplying and extracting air comprises a closing valve (6) which is provided on the receptacle (2) and can be connected to a vacuum and air pressure pump.

11. The extension device in accordance with claim 1 **characterised in that** a coupling element, which can be coupled to tensioning device (8), is provided on the side of the receptacle (2) which is opposite the opening (3).

12. The extension device in accordance with claim 11 **characterised in that** the coupling element is integrated into the closing valve (6).

13. The extension device in accordance with claim 1 **characterised in that** the tensioning device (8) is formed of a carrier, strap, belt or other clothing accessory or item of clothing connected to the receptacle (2), held on the body of the user and acting as a an abutment, or also a stationary object or longitudinally-adjustable system of rods supported on the body or a weight suspended on the receptacle.

## Revendications

1. Appareil d'extension pour l'agrandissement et la rectification durables du pénis par une extension de longue durée, comprenant un moyen de fixation à placer sur le pénis et un dispositif de traction relié à celui-ci, **caractérisé en ce que** le moyen de fixation présente un corps de réception (2) indéformable recevant dans une large mesure toute la surface du gland du pénis par complémentarité de forme, dont le contour intérieur correspond dans une large mesure à la forme du gland du pénis, *sachant que selon* la forme *et la taille du gland du pénis, des corps de réception (2) de* conceptions différentes *sont prévus,* et lequel peut être relié à un moyen pour transporter et aspirer de l'air afin de produire une surpression et une dépression et sur l'ouverture (3) duquel, prévue pour introduire le gland du pénis, se raccorde un joint tubulaire souple élastique (15).

2. Appareil d'extension pour l'agrandissement et la rectification durables du pénis par une extension de longue durée, comprenant un moyen de fixation à placer sur le pénis et un dispositif de traction relié à celui-ci, **caractérisé en ce que** le moyen de fixation présente un corps de réception (2) indéformable recevant dans une large mesure toute la surface du gland du pénis par complémentarité de forme, dont le contour intérieur correspond dans une large mesure à la forme du gland du pénis, *sachant que selon la forme et la taille respectives du gland du pénis, des inserts de conceptions différentes peuvent être intégrés dans le corps de réception (2), lesquels sont formés en étant adaptés ou en s'adaptant automatiquement à la face intérieure du corps de réception et au contour extérieur du gland du pénis,* et lequel peut être relié à un moyen pour transporter et aspirer de l'air afin de produire une surpression et une dépression et sur l'ouverture (3) duquel, prévue pour introduire le gland du pénis, se raccorde un joint tubulaire souple élastique (15).

3. Appareil d'extension pour l'agrandissement et la rectification durables du pénis par une extension de longue durée, comprenant un moyen de fixation à placer sur le pénis et un dispositif de traction relié à celui-ci, **caractérisé en ce que** le moyen de fixation présente un corps de réception (2) recevant amplement toute la surface du gland du pénis par complémentarité de forme, dont le contour intérieur correspond dans une large mesure à la forme du gland du pénis, ***en ce que** au-dessus de l'ouverture (3) du corps de réception (2), est appliquée une membrane (18) élastique pouvant être bombée vers l'extérieur sous l'effet d'une surpression et pouvant être invaginée sous l'effet d'une dépression lors de l'introduction du gland du pénis dans le corps de réception, laquelle entoure dans une large mesure toute la surface du gland du pénis par complémentarité de forme, indépendamment de ses forme et taille respectives,* et lequel peut être relié à un moyen pour transporter et aspirer de l'air afin de produire une surpression et une dépression et sur l'ouverture (3) duquel, prévue pour introduire le gland du pénis, se raccorde un joint tubulaire souple élastique (15).

4. Appareil d'extension selon l'une des revendications 1 à 3, **caractérisé en ce que** dans l'état d'utilisation, un lubrifiant est présent sur la face intérieure du corps de réception (2) ou de l'insert ou de la membrane (18) pour favoriser la liaison intérieure agissant de manière adhésive avec le gland du pénis.

5. Appareil d'extension selon la revendication 1, **caractérisé en ce qu'**un lubrifiant est prévu sur la face intérieure du joint tubulaire (15) pour faciliter l'introduction du gland du pénis.

6. Appareil d'extension selon la revendication 5, **caractérisé en ce que** l'extrémité libre du joint tubulaire (15) éloignée du ccrps de réception (2) est reliée à un anneau de soutien (17) dimensionné en correspondant environ au diamètre du pénis, pour une introduction facile du gland du pénis.

7. Appareil d'extension selon la revendication 1 ou 6, **caractérisé en ce que** le joint tubulaire (15) est fixé de manière élastique aux extrémités dans une rainure périphérique (16) de l'anneau de soutien (17) et dans une rainure de fixation (14) formée sur le corps de réception (2).

8. Appareil d'extension selon la revendication 7, **caractérisé en ce que** le joint tubulaire (15) présente un bourrelet de fixation élastique sur au moins une extrémité.

9. Appareil d'extension selon la revendication 1, **caractérisé en ce que** le joint tubulaire (15) est formé par un tronçon de douille et la membrane (18) est formée par un tronçon de ballon d'un ballon gonflable du commerce de taille correspondante invaginé au-dessus du corps de réception (2).

10. Appareil d'extension selon la revendication 1, **caractérisé en ce que** le moyen pour transporter et aspirer de l'air comprend une soupape de fermeture (6) prévue sur le corps de réception (2), à laquelle une pompe d'aspiration et de refoulement de l'air peut être raccordée.

11. Appareil d'extension selon la revendication 1, **caractérisé en ce qu'**un élément de couplage est prévu sur le côté du corps de réception (2) opposé à l'ouverture (3), lequel peut être couplé à un dispositif de traction (8).

12. Appareil d'extension selon la revendication 11, **caractérisé en ce que** l'élément de couplage est intégré dans la soupape de fermeture (6).

13. Appareil d'extension selon la revendication 1, **caractérisé en ce que** le dispositif de traction (8) est formé par un support, une ceinture, un ceinturon ou un accessoire d'habillement divers ou par une pièce d'habillement maintenue sur le corps de l'utilisation ou servant de contre-appui, ou aussi par un objet fixe ou des tiges réglables en longueur appuyées sur le corps ou par un poids suspendu sur le corps de réception (2).
